Europäisches Patentamt

European Patent Office

Office européen des brevets

(19)

(11) EP 0 745 609 B1

(12) EUROPÄISCHE PATENTSCHRIFT

(45) Veröffentlichungstag und Bekanntmachung des Hinweises auf die Patenterteilung:
**02.02.2000 Patentblatt 2000/05**

(51) Int Cl.⁷: **C07K 14/155**, C07K 14/16, A61K 39/21

(21) Anmeldenummer: **96108816.8**

(22) Anmeldetag: **01.06.1996**

(54) **Vakzine zur Prophylaxe gegen eine HIV-Infektion und/oder zur Therapie von AIDS, und Verfahren zur Herstellung**

Vaccines for prophylaxis against HIV infections and/or for AIDS therapy and process for preparing them

Vaccins pour la prophylaxie contre des infections par le virus VIH et/ou pour le traitement du SIDA et procédé de préparation

(84) Benannte Vertragsstaaten:
**AT BE CH DE ES FR GB IE IT LI NL PT SE**

(30) Priorität: **01.06.1995 DE 19520216**

(43) Veröffentlichungstag der Anmeldung:
**04.12.1996 Patentblatt 1996/49**

(73) Patentinhaber:
• **Frey, Rainer H.**
  **82319 Starnberg (DE)**
• **Shtengold, Efim S.**
  **117526 Moskau (RU)**

(72) Erfinder:
• **Sergeyev, Oleg V.**
  **117571 Moskau (RU)**
• **Shtengold, Efim S.**
  **117526 Moskau (RU)**
• **Garaev, Mansur M.**
  **129560 Moskau (RU)**
• **Godin, Eugene A., Dr.**
  **129164 Moskau (RU)**

(74) Vertreter: **Brehm, Hans-Peter, Dr. Dipl.-Chem.**
  **Patentanwälte Kern, Brehm & Partner GbR**
  **Albert-Rosshaupter-Strasse 73**
  **81369 München (DE)**

(56) Entgegenhaltungen:
  **EP-A- 0 514 199**          **WO-A-91/05860**
  **US-A- 4 775 625**

• **THE JOURNAL OF GENERAL PHYSIOLOGY, Bd. 34, Nr. 3, 20.Januar 1951, USA, Seiten 305-319, XP000196346 A. D. HERSHEY ET AL: "The mortality of bacteriophage containing assimilated radioactive phosphorus"**

**Beschreibung**

Die vorliegende Erfindung betrifft eine Vakzine, welche das inaktivierte Genom von Viren enthält, hier insbesondere von Retroviren und hier insbesondere das inaktivierte Genom von HIV. Diese Vakzine ist brauchbar zur Prophylaxe gegen eine HIV-Infektion und/oder zur Therapie von AIDS.

Bekanntlich liegt der Impfung mit einer Vakzine das Konzept zugrunde, eine Immunreaktion des Organismus zu stimulieren und gegebenenfalls zu verstärken. Ein wichtiger Aspekt dieser Immunreaktion ist die Bildung von Antikörpern. Die Bildung von Antikörpern wird durch die Anwesenheit von Antigenen provoziert. Hierbei handelt es sich um geschwächte oder völlig inaktivierte Erreger, welche die zu behandelnde Krankheit verursachen. Eine Prophylaxe gegen eine HIV-Infektion und/oder eine Behandlung von AIDS durch Verabreichung einer Vakzine, welche das inaktivierte Gesamtgenom von HIV-Partikeln enthält, ist aus mehreren Gründen problematisch. Wegen der hohen Virulenz des HIV muß die sichere Inaktivierung des Virus gewährleistet sein, um eine gefahrlos verabreichbare Vakzine bereitzustellen. Andererseits dürfen die Maßnahmen zur Inaktivierung die antigenen Eigenschaften des Virus nicht übermäßig verändern, da sonst die Provozierung einer Bildung der richtigen Antikörper beeinträchtigt ist.

Jede einzelne Genom-Kopie der HIV-Partikel besteht aus einem RNA-Strang, der etwa 9.500 Nukleotide aufweist. Die geringe Länge dieses RNA-Stranges im Vergleich zur Oberfläche der Viruspartikel erfordert hohe Intensitäten von UV- oder Röntgenstrahlung, um diese RNA sicher zu inaktivieren. Andererseits verändert eine hohe Strahlungsdosis die antigenen Eigenschaften der Virushülle. Die RNA von HIV ist wenigstens bis zu Temperaturen von 55 bis 60°C beständig; im Einzelfall wurde sogar eine Beständigkeit bis 100°C berichtet. Auch gegenüber organischen Detergentien, Alkohol, Aceton, Formalin, Phenol und anderen Substanzen scheint zumindest die RNA der HIV-Partikel beständig zu sein.

Die derzeit wohl fortschrittlichste Vakzine zur Therapie von HIV scheint "Salk's HIV-Immunogen" zu sein (vgl. Proc. Natl. Acad. Sci. USA, Band 88, S. 3348-3352, April 1991). Hier werden Partikel von HIV-1 mit Beta-Propiolacton und Gamma-Strahlung inaktiviert; jedoch fehlt am inaktivierten Material das Glykoprotein gpl20. Aus diesem Grunde sind die immunogenen Eigenschaften dieses Präparates beeinträchtigt. Es besteht weiterhin Bedarf nach einer Vakzine zur Prophylaxe gegen eine HIV-Infektion und/oder zur Therapie von AIDS und anderer durch Retroviren verusachten Krankheiten, deren aktive Komponenten einerseits sicher inkaktiviert sind und andererseits hohe antigene Wirksamkeit aufweisen.

Bereits 1951 war die Möglichkeit einer Virusinaktivierung durch den radioaktiven Zerfall von $^{32}_{15}$ P demonstriert worden, der in das DNA-Genom von T2- und T4-Phagen eingebaut worden war; vergleiche: J. Gen. Physiol. 34, 305-319 (1951). Die Autoren konnten zeigen, daß das Ausmaß der Inaktivierung mit der Zerfallskonstante von $^{32}_{15}$ P korreliert.

Das technische Problem der vorliegenden Erfindung besteht darin, eine Vakzine zur Prophylaxe gegen eine Infektion durch HIV und andere Retroviren und/oder zur Therapie von AIDS und/oder anderer durch Retroviren verusachte Krankheiten bereitzustellen, deren aktive Komponente(n) sicher inaktiviert ist, wobei der inaktivierte Virus dennoch hohe antigene Aktivität aufweist.

Zur Lösung dieses technischen Problems wird mit der vorliegenden Erfindung eine Vakzine zur Prophylaxe gegen eine HIV-Infektion und/oder zur Therapie von AIDS, mit einem Gehalt an inaktiviertem HIV-Genom vorgeschlagen. Diese Vakzine ist dadurch gekennzeichnet, daß das inaktivierte HIV-Genom erhältlich ist durch

- Kultivierung von HIV in einem Medium, das als Phosphorquelle das radioaktive Phosphorisotop $^{32}_{15}$ P ententhält;
- Isolierung und Abtrennung der kultivierten HIV-Partikel aus dem Kulturmedium unter Bedingungen, die wenigstens 50 % oder mehr des viralen gp120-Antigens erhalten; und
- Aufbewahrung der so gebildeten und isolierten HIV-Partikel, deren RNA wenigstens eine Phosphorsäure-Di-Estergruppe enthält, deren Phosphorspezies aus $^{32}_{15}$ P besteht für einen Zeitraum von mehr als zwei Monate bei einer Temperatur von minus 70°C oder unterhalb minus 70°C.

Nach einer bevorzugten Ausführungsform der Erfindung wird das inaktivierte HIV-Genom unter solchen Bedingungen erhalten, die gewährleisten, daß jede einzelne Genom-RNA wenigstens 1 bis 4 radioaktive Phosphorisotope $^{32}_{15}$ P aufnimmt.

Eine solche Aufnahme von radioaktivem Phosphor und Einbau in die Virus-RNA gewährleistet eine vollständige Inaktivierung des Virus.

Nach einer weiteren vorteilhaften Ausgestaltung der Erfindung handelt es sich bei den inaktivierten HIV-Partikeln, die nach der Lang-Zeit-Tief-Temperatur-Aufbewahrung erhalten werden, um ein Gesamt-Virion des Virus. Dieses inaktivierte Gesamt-Virion gewährleistet die Gesamtheit der immunogenen Eigenschaften des ursprünglichen Virus. Diese Lang-Zeit-Tief-Temperatur-Aufbewahrung vonmehr als zwei Monaten gewährleistet, daß der größte Anteil an eingebautem, radioaktivem Phosphorisotop $^{32}_{15}$ P in der Zwischenzeit zerfallen ist, so daß ein Präparat erhalten wird, das in radioaktiver Hinsicht maximale Sicherheit verspricht.

In der erfindungsgemäßen Vakzine sind die HIV-Partikel spezifisch, vollständig und irreversibel inaktiviert. Das Präparat weist keinerlei infektiöse Eigenschaften auf. Dennoch ist die antigene Aktivität der Viruspartikel maximal erhalten und gewährleistet. Eine Besonderheit besteht darin, daß die Inaktivierung der Genomstruktur von innen heraus erfolgt, ohne die Hülle der Viruspartikel zu beschädigen, von welcher im wesentlichen die antigenen Eigenschaften ausgehen. Bei der erfindungsgemäßen Form der Inaktiverung können eine Anzahl verschiedener Virusvarianten, die einen gewissen Polymorphismus ihrer Genomstruktur aufweisen, gleichzeitig inaktiviert werden, so daß ein breites Spektrum antigener Eigenschaften erhalten wird. Weiterhin kann die Provozierung einer bestimmten Immunantwort, die unterschiedlich sein kann im Falle einer Prophylaxe gegen eine HIV-Infektion oder im Falle einer Therapie von AIDS, beeinflußt werden durch adäquate Dosierung und den zeitlichen Verlauf der Verabreichung. Diese erfindungsgemäße Vakzine erfüllt die Anforderungen, die an eine heutige und fortschrittliche Strategie zur Immunisierung und Impfung gestellt werden, wobei sowohl eine durch die verabreichten Zellen und deren Bestandteile und Wirkstoffe verursachte Immunisierung wie eine durch die erzeugten Antikörper bewirkte Immunantwort hervorgerufen wird (vgl. die so-genannten Salk's Criteria in der Veröffentlichung "Strategy for Prophylactic Vaccination Against HIV" von J. Salk, P.A. Bretscher, P.L. Salk, M. Clevici und G.M. Shearer in Science, Band 260, S. 1270-1272, 1993).

Weiterhin betrifft die Erfindung ein Verfahren zur Herstellung einer Vakzine zur Prophylaxe gegen eine HIV-Infektion und/oder zur Therapie von AIDS. Dieses Verfahren ist gekennzeichnet durch nachstehende Verfahrensschritte:

- aktive HIV-Partikel werden in einem Kulturmedium vermehrt, das als Phosphorquelle das radioaktive Phosphorisotop $^{32}_{15}$ P enthält;
- die so gebildeten HIV-Partikel werden aus dem Kulturmedium unter Bedingungen abgetrennt und isoliert, die wenigstens 50 % oder mehr des viralen gp120-Antigens erhalten; und
- die so isolierten HIV-Partikel, deren RNA wenigstens eine Phosphorsäure-Di-Estergruppe enthält, deren Phosphorspezies aus $^{32}_{15}$ P besteht, werden für einen Zeitraum von mehr als zwei Monate bei einer Temperatur von minus 70°C oder unterhalb minus 70°C aufbewahrt; und
- daraufhin werden diese HIV-Partikel zu einer applizierbaren Vakzine konfektioniert.

Vorteilhafte Ausgestaltungen und Weiterbildungen der Erfindung ergeben sich aus den Unteransprüchen.

Bekanntlich weisen die Ribonukleinsäuren eine Kettenstruktur mit einer Zucker-Phosphat-Kette auf, wobei die Nukleotide als Seitenketten an Ribose gebunden sind. Je eine Phosphorsäuregruppe bildet als Di-Ester das Bindeglied zwischen zwei Ribosemolekülen. Weiterhin ist bekannt, daß durch Neutronenbeschuß von normalem Phosphor $^{32}_{15}$ P radioaktiver Phosphor $^{32}_{15}$ P erhältlich ist, der nach der Gleichung

$$^{32}_{15}\text{P} \rightarrow\ ^{32}_{16}\text{S} +\ ^{0}_{-1}\text{e} + \gamma$$

mit einer Halbwertszeit von 14,22 Tagen in normalen Schwefel $^{32}_{16}$ S zerfällt. Dieser radioaktive Phosphor steht in Form von entsprechend angereicherter Orthophosphorsäure zumindest für wissenschaftliche Zwecke handelsüblich zur Verfügung.

Die vorliegende Erfindung geht von der Überlegung aus, daß ein Austausch des normalen Phosphors $^{31}_{15}$ P in der Zucker-Phosphat-Kette der RNA der Viruspartikel durch radioaktiven Phosphor $^{32}_{15}$ P über den radioaktiven Zerfall zur Bildung von $^{32}_{16}$ S führt. Der so gebildete Schwefel kann die Verknüpfung der Ribosemoleküle nicht länger gewährleisten. Weiterhin kann die beim radioaktiven Zerfall von $^{32}_{15}$ P auftretende Elektronenstrahlung (Betastrahlung) und γ-Strahlung ebenfalls zu einer Schädigung der Ribonukleinsäuren beitragen. Diese zuletzt genannten Wirkungen können solche RNA-Kopien verändern, die im Verlauf ihrer Synthese kein $^{32}_{15}$ P aufgenommen haben. Dieser Mechanismus scheint die Inaktivierung in solchen Fällen hervorzurufen, wo ein einzelner RNR-Strang im Mittel weniger als ein $^{32}_{15}$ P-Isotop aufgenommen hat und die Konzentration an Viruspartikeln ausreichend hoch ist.

Damit sollte eine Inaktivierung der RNA-Struktur zu erreichen sein, ohne die antigenen Eigenschaften der Viruspartikel zu beeinträchtigen, die vor allem auf die Hüllenproteine und die "Knospen" zurückzuführen sind, welche über die Lipidhülle vorstehen.

Über diese allgemeinen Betrachtungen hinaus bringt die vorliegende Erfindung, die zusätzliche, neue und überraschende Lehre, daß der RNA-Strang nach einer ausreichenden Aufnahme und Einbau von $^{32}_{15}$ P für eine längere Zeitspanne (die von der Menge an $^{32}_{15}$ P abhängig ist; zur Dauer dieser Zeitspanne erfolgt nachstehend eine ausführliche Berechnung) bei tiefer Temperatur gehalten werden muß, um eine erneute Rekombination von Viruskomponenten zu verhindern, und um eine vollständige und sichere Inaktivierung zu gewährleisten.

Die Weiterverarbeitung bzw. Konfektionierung des nach der Lang-Zeit-Tief-Temperatur-Aufbewahrung erhaltenen inaktivierten HIV-Genomes zu der applizierbaren Vakzine kann nach üblichen Verfahren erfolgen, die in der Fachwelt gut bekannt sind. Die so erhaltene therapeutische anti-HIV-Vaccine kann an mit HIV infizierte Patienten verabreicht werden (Impfung nach einer Ansteckung). Weiterhin können die inaktivierten Viruspartikel zur Herstellung von prophylaktischen Impf-Präparaten verwendet werden, deren Verabreichung an die Mitglieder verschiedener Risikogruppen vorgeschlagen wird.

Die nachstehenden Ausführungen dienen zur weiteren Erläuterung und Ausgestaltung der Erfindung. Die Zeichnungen zeigen:

Figur 1    für die verschiedenen Ansätze "a", "b" und "c" und für einen zusätzlichen Kontrollansatz "d" die Verteilung der ermittelten Radioaktivität (CpM = Counts per Minute) auf die verschiedenen Fraktionen; ersichtlich weisen die Fraktionen 3 bis 6 jeweils die höchste Radioaktivität auf;

Figur 2    die zeitabhängige Abnahme der relativen Virusinfektiosität der verschiedenen Ansätze "a" bis "d" während einer Lagerung bei +4°C;

Figur 3    die zeitabhängige Abnahme der relativen Virusinfektiosität der Kontrollprobe "d" bei verschiedenen Temperaturen; nämlich bei +4°C (ausgezogene Linie), bei minus 70°C (gestrichelte Linie) und bei minus 170°C (punktierte Linie);

Figur 4    die zeitabhängige Abnahme der relativen Virusinfektiosiät der verschiedenen Ansätze "a" bis "d" während einer Lagerung bei minus 70°C; und

Figur 5    die zeitabhängige Abnahme der relativen Virusinfektiosität der verschiedenen Ansätze "a" bis "d" während einer Lagerung bei minus 170°C.

Lymphoide CEM-SS-Zellen werden in einem strömenden RPMI-1640-Wachstumsmedium gezüchtet, das 10 % fetales Kälberserum und 0,03 % Glutamin enthält. Die Züchtung erfolgt bei 37°C unter einer Atmosphäre, die 5 % $CO_2$ enthält. Als Virusquelle dient der Stamm HIV-1-RF. Die Zellen werden mit diesem Virus infiziert bei einer Dosis von einer Syncytium-bildenden Einheit (SFU) auf 50 bis 100 CEM-SS-Zellen. Einen Tag vor der Virusinfektion wird das Wachstumsmedium gegen ein solches RPMI-1640-Medium ausgetauscht, das einen 10-fach geringeren Gehalt an Phospaten aufweist. Zwei Stunden nach der Virusinfektion wird der Kultursuspension Orthophosphorsäure zugesetzt, die mit radioaktivem Phosphor ($^{32}_{15}$ P) angereichert ist.

Im einzelnen wird die Kultursuspension auf 4 Ansätze (a) bis (d) aufgeteilt. Dem Ansatz (a) wird eine solche Menge an radioaktivem Phosphor $^{32}_{15}$ P zugesetzt, um eine Aktivität von 5,2 µCi (Mikro-Curie) pro 1 ml Kulturmedium zu erzielen; dies ergibt ein Verhältnis von 1 radioaktves Phosphorisotop $^{32}_{15}$ P auf 300.000 nicht-radioaktive Phosphorisotope. Dem Ansatz (b) wird eine solche Menge radioaktiver Phosphor $^{32}_{15}$ P zugesetzt, um eine Aktivität von 46,8 µCi pro 1 ml Kulturmedium zu erzielen; dies entspricht einem Verhältnis von 1 radioaktives Phosphorisotop auf 30.000 nicht-radioaktive Phosphorisotope. Dem Ansatz (c) wird eine solche Menge radioaktiver Phosphor $^{32}_{15}$ P zugesetzt, um eine Aktivität von 156,0 µCi pro 1 ml Kulturmedium zu erzielen; das entspricht einem Verhältnis von 1 radioaktivem Phosphorisotop auf 10.000 nicht-radioaktive Phosphorisotope. Der Ansatz (d) wird mit herkömmlicher, nicht-radioaktiver Orthophosphorsäure versetzt und dient als Kontrollprobe. Die Züchtung von HIV wird für 5 aufeinanderfolgende Tage fortgesetzt. Die so gezüchteten Viren werden gewonnen am 5. Tag nach der Infektion bei einer maximalen Ausbeute der reifen Virions.

Diese Ansätze (a) bis (d) werden 5 Tage lang unter Wachstumsbedingungen für das Virus gehalten. Anschließend wird das Kulturmedium geklärt. Aus jedem Ansatz werden je 12 Anteile von je 100 µl Virus enthaltender Flüssigkeit gewonnen, an denen nach der Lang-Zeit-Tief-Temperatur-Aufbewahrung die Virusinfektiosität bestimmt wird. Diese Anteile werden mit PEG 6000 und Dextran nach bekannten Maßnahmen behandelt, um ein Viruskonzentrat zu erhalten (AIDS Res. Human Retrovir., 1990, Band 6, Seite 1379). Um nachzuweisen, daß die virale RNA radioaktiven Phosphor $^{32}_{15}$ P aufgenommen hat, werden die Virus enthaltenden Konzentrate in einem Sucrosegradienten (5 bis 60 %) bei 150.000 g 2 Stunden lang zentrifugiert. Daraufhin wird jede so erhaltene Probe in 20 Fraktionen von je 200 µl unterteilt.

15 µl jeder Fraktion werden auf ein Papierfilter gegeben. Zur Entfernung löslicher Komponenten wird jedes Papierfilter mit 5 %-iger Trichloressigsäure von 4°C ausgewaschen. Die getrockneten Filter werden in Toluol Mit Hilfe eines Hewlett-Packard-Counters ausgewertet. Für die verschiedenen Ansätze "a", "b" und "c" sind die Spitzenwerte der Radioaktivität jeweils in den Fraktionen mit der gleichen Dichte gefunden worden, nämlich bei einer Dichte von 1,167 g/ml; das entspricht der Dichte des gesamten HIV-Virions. Der Einbau bzw. die Aufnahme von $^{32}_{15}$ P wird berechnet auf der Basis der Anzahl/Menge der Viruspartikel, die in den Fraktionen mit der höchsten Radioaktivität vorhanden sind. Hierbei wird für die nachfolgende Berechnung angenommen, daß die, die Radioaktivität verursachenden $^{32}_{15}$ P-Isotope zu 100 % in die Genom-RNA eingebaut worden sind.

Die Anzahl der Viruspartikel wird bestimmt mit Hilfe des ELISA-Prüfverfahrens unter Verwendung monoklonaler Antikörper zum p24-Antigen des Virus. Als Referenz dienen die optischen Dichten von Standardlösungen mit bekannten Konzentrationen an p24-Antigen. Als kleinstes positives Signal dient eine optische Dichte von 0,120 bei der Wellenlänge 492 nm, was einer p24-Konzentration von 0,25 ng/ml entspricht.

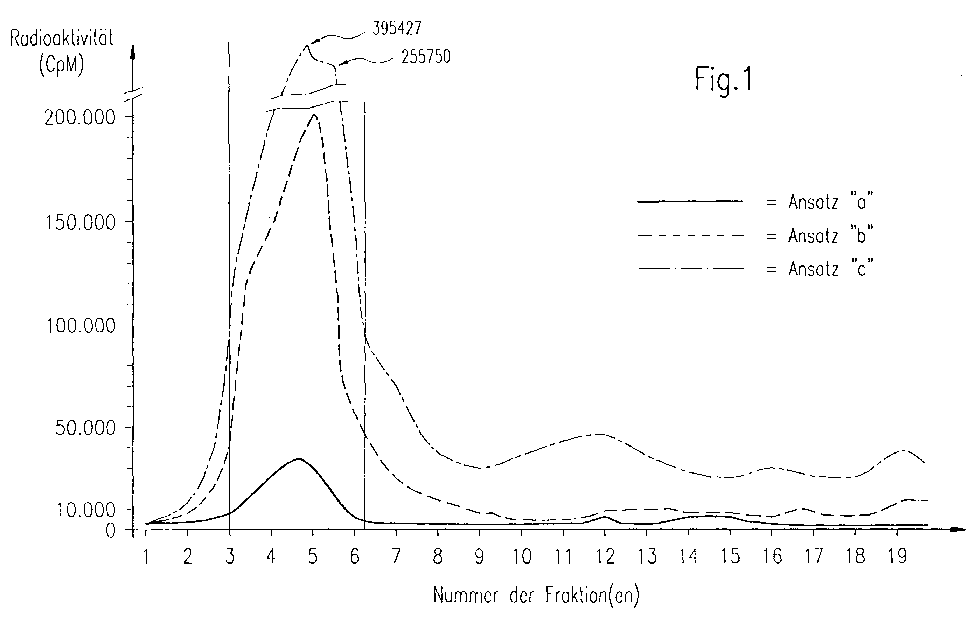

Bei allen drei Ansätzen (a) bis (c) weisen jeweils die Fraktionen 3 bis 6 die höchste Radioaktivität auf. Die Verteilung der Radioaktivität auf die Fraktionen der Ansätze ist aus Fig. 1 ersichtlich. 600 µl dieser vereinigten Fraktionen aus jedem Ansatz enthalten 7.200 ng p24. Aus jedem Ansatz werden aus den Fraktionen 4, 5 und 6 je 45 µl (15 x 3) bereitgestellt und daran die Radioaktivität geprüft. Die Ergebnisse sind in der nachstehenden Tabelle 1 dargestellt.

TABELLE 1

| Radioaktivität der Virion-RNA in Abhängigkeit von der Konzentration an radioaktivem Phosphor im Kulturmedium | | | | |
|---|---|---|---|---|
| Verhältnis $^{32}_{15}$ P : $^{31}_{15}$ P im Medium | Verhältnis von $^{32}_{15}$ P zur Anzahl der RNA-Kopien | Zählimpulse (cpm) in den Fraktionen 4,5,6 (Dichte ca. 1,167 g/ml) | | Summe der Zählimpulse (cpm) in den Fraktionen 4, 5 und 6 |
| 1 : 300 00 | 1 : 30 | 4 | 24.633 | 67.460 |
| | | 5 | 32.078 | |
| | | 6 | 10.749 | |
| 1 : 30 000 | 1 : 3 | 4 | 121.259 | 389.349 |
| | | 5 | 198.081 | |
| | | 6 | 70.009 | |
| 1 : 10 000 | 1 : 1 | 4 | 134.748 | 785.925 |
| | | 5 | 395.427 | |
| | | 6 | 255.750 | |

Die vereinigten Fraktionen "4", "5" und "6" aus dem Kontrollansatz "d" weisen eine Konzentration an p24-Antigen entsprechend 12 µg/l auf. Die vereinigten Fraktionen "1" bis "3" und "7" bis "20" des Kontrollansatzes "d" weisen eine Konzentration an p24-Antigen entsprechend 0,86 µg/l auf.

Ersichtlich weisen die vereinigten Fraktionen "4", "5" und "6" aus dem Ansatz "a", der radioaktiven Phosphor $^{32}_{15}$ P in einer Konzentration von 5,2 µCi/ml Kulturmedium enthält, 67.460 gezählte Impulse auf. Entsprechend weisen die vereinigten Fraktionen "4", "5" und "6" aus dem Ansatz "b", der radioaktiven Phosphor $^{32}_{15}$ P in einer Konzentration von 46,8 µCi/ml Kulturmedium enthält, 389.349 gezählte Impulse auf. Die vereinigten Fraktionen "4", "5" und "6" aus dem Ansatz "c", der radioaktiven Phosphor $^{32}_{15}$ P in einer Konzentration von 156,0 µCi/ml Kulturmedium enthält, weisen 785.925 gezählte Impulse auf.

Diese Ergebnisse bestätigen, daß die Aufnahme von radioaktivem Phosphor $^{32}_{15}$ P im Verlauf der RNA-Synthese ausschließlich vom Verhältnis der Konzentrationen von radioaktivem Phosphor zu nicht-radioaktivem Phosphor im Kulturmedium abhängt.

Die aufgenommene Radioaktivität und die gemessenen Konzentrationen des p24-Antigens bestätigen, daß die $^{32}_{15}$ P-Isotope nahezu vollständig in die Viruspartikel eingebaut worden sind, das heißt in das RNA-Genom des Virus. Da jedes einzelne HIV-Genom im wesentlichen 9.500 Nukleotide aufweist, kommt ein eingebautes $^{32}_{15}$ P-Isotop

- in dem Ansatz "a" auf etwa 30 Genom-Kopien,
- in dem Ansatz "b" auf etwa 3 Genom-Kopien,
- in dem Ansatz "c" auf etwa 1 Genom-Kopie.

Bekanntlich zerfällt das radioaktive Phosphorisotop $^{32}_{15}$ P unter Abgabe von Beta-Strahlung und Gamma-Strahlung in das nicht-radioaktive Schwefelisotop $^{32}_{16}$ S. Schwefel kann die 5-Bindigkeit des Phosphors der Phoshporsäure-Di-Estergruppe in der Zucker-Phosphat-Kett der RNA-Struktur nicht länger gewährleisten. Hinzu kommt eine mögliche Schädigung der Umgebung des eingebauten $^{32}_{15}$ P-Isotops durch die abgegebene Strahlung. Deshalb kommt es beim radioaktiven Zerfall von $^{32}_{15}$ P zu $^{32}_{16}$ S zu Veränderungen der RNA-Struktur, die mit einer Abnahme der Virusinfektiosität verbunden ist. Im Rahmen der Erfindung wurde erkannt, daß die in der Praxis tatsächlich erzielbare Abnahme der Virusinfektiosität abhängig ist von Dauer und Temperatur bei einer Aufbewahrung der $^{32}_{15}$ P enthaltenden Genom-RNA. Zumindest während der wesentlichen Phase des radioaktiven Zerfalls muß das Präparat unterhalb bestimmter Temperaturen gehalten werden, um zu einer dauerhaften und sicheren Abnahme der Virusinfektionsität zu kommen.

Dieses Ergebnis wird mit den nachfolgenden Untersuchungen bestätigt.

Die Virusinfektiosität wird anhand von in vitro-Untersuchungen zur Synzytien-Bildung und zur p24-Synthese geprüft. Am Tag "Null" wird die Virusinfektiosität zu 100 % angenommen (dies entspricht dem dekadischen Logarithmus lg = 2). Die Virus enthaltenden Flüssigkeitsanteile werden auf das 3-fache, 9-fache, 27-fache, ... 59.049-fache Volumen verdünnt. Aus jeder Verdünnung werden 50 µl zu 100 µl CEM-SS-Zellensuspension hinzugefügt; jeder Versuch wird in vier gleichen Ansätzen durchgeführt. Das Gemisch aus Zellen mit Virus wird in Platten mit 96 Näpfen inkubiert. Nach 10 Tagen wird die Entwicklung anhand der Sycythia-Bildung bestimmt. Am 10. Tag wird die Anzahl/Menge der Viruspartikel mit Hilfe von ELISA zum p24-Antigen bestimmt. Die letzten Verdünnungen, in denen noch neu synthetisierten p24-Antigen nachweisbar waren, werden als Titer der Infektiosität herangezogen.

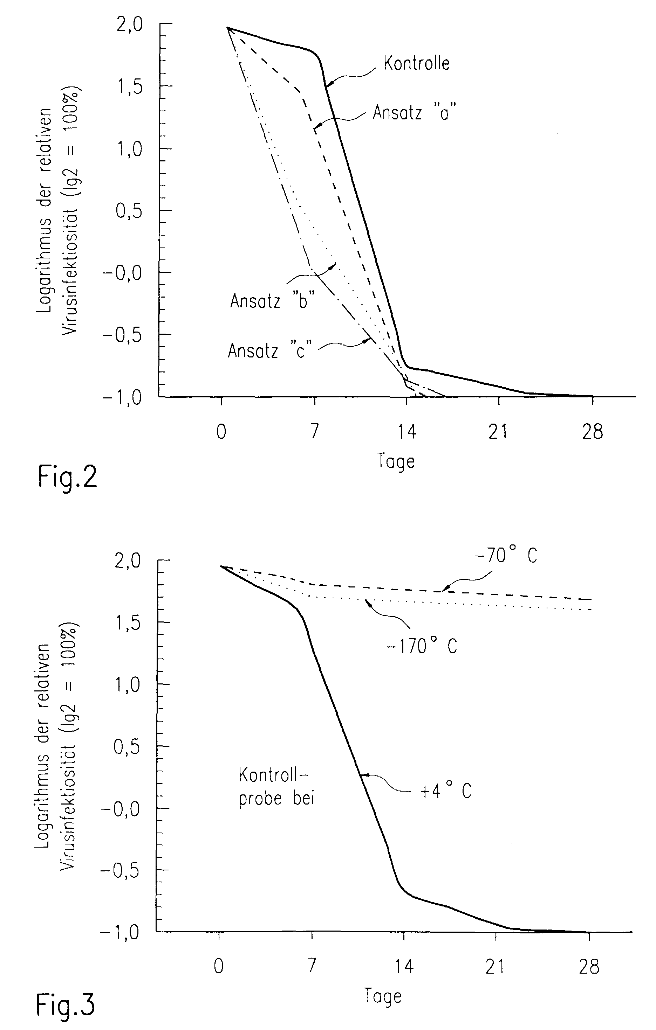

In einer ersten Versuchsreihe werden die Ansätze "a" bis "d" 30 Tage lang bei +4°C erhalten. Die hierbei ermittelte Abnahme der Virusinfektiosität ist graphisch mit Figur 2 dargestellt. Die Ergebnisse zeigen, daß wohl anfänglich die Infektiosität der radioaktiven Proben schneller abnimmt als die Infektiosität der Kontrollprobe "d" abnimmt. Auch scheint die Abnahme der Infektiosität der radioaktiven Proben abhängig zu sein vom Gehalt an radioaktivem Phosphor $^{32}_{15}$ P. Jedoch läßt sich Ablauf von 14 Tagen kein nennenswerter Unterschied in der Infektiosität der verschiedenen Proben feststellen.

Aliquote Anteile des Kontrollsatzes "d" sind 28 Tage lang bei +4°C, bei minus 70°C und bei minus 170°C gehalten worden. Die Figur 3 zeigt die Abnahme der Infektiosität der Kontrollprobe "d" während der Aufbewahrung bei diesen Temperaturen. Ersichtlich wird bei einer Aufbewahrung bei +4°C wieder die unspezifische Abnahme der Virusinfektiosität erhalten, die bereits aus Figur 2 ersichtlich ist. Jedoch kann bei einer Aufbewahrung dieser Kontrollprobe "d" bei minus 70°C oder minus 170°C diese Abnahme der Infektiosität im wesentlichen verhindert werden. Offensichtlich werden bei diesen tiefen Temperaturen die Stoffwechselvorgänge so weit verlangsamt oder "eingefroren" daß es nach einer anfänglichen geringfügigen Abnahme im wesentlichen zu einem stabilen Zustand kommt. Dies deckt sich auch mit anderen Erfahrungen, wonach die Aktivität von HIV-Partikeln durch Aufbewahrung bei tiefen Temperaturen im wesentlichen nicht beseitigbar ist. Werden solche Viruspartikel erneut in eine zur Vermehrung geeignete Umgebung gebracht, so kehrt auch die Infektiosität der Viruspartikel zurück.

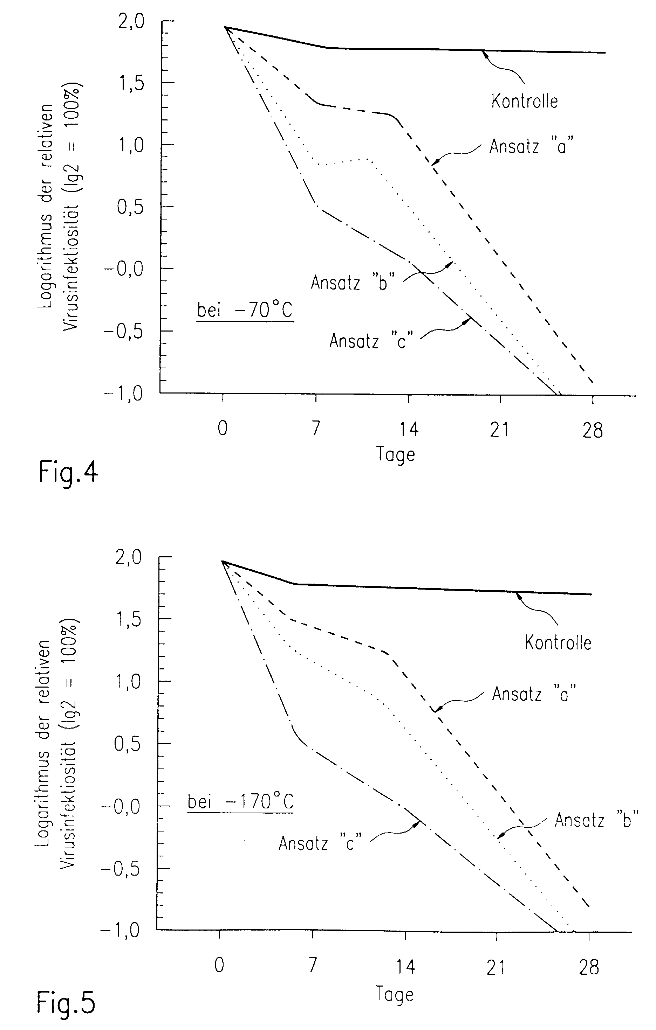

In einer weiteren Versuchsreihe werden sämtliche Ansätze "a" bis "d" bei minus 70°C gehalten. Die dabei festgestellte Abnahme der Virusaktivität ist graphisch mit Figur 4 dargestellt. Ersichtlich bleibt die Aktivität der Kontrollprobe "d" nach einer anfänglichen geringfügigen Abnahme im wesentlichen konstant, wie das bereits mit Figur 3 nachgewiesen ist. Demgegenüber nimmt jedoch die Aktivität der radioaktiven Proben weiter ab. Diese Abnahme der Aktivität ist deutlich abhängig vom Gehalt an radioaktivem Phosphor in jeder Probe, und beruht letztlich auf dem Zerfall von $^{32}_{15}$ P.

Die Figur 5 bestätigt diese Ergebnisse für die Aufbewahrung aller Proben bei minus 170°C.

In der nachstehenden Tabelle 2 sind die Meßwerte aufgeführt, die zu den vorstehend referierten Ergebnissen geführt haben.

Tabelle 2

| Abnahme der HIV-Infektiosität in Abhängigkeit von, der 32-P-Konzentration, der Aufbewahrungstemperatur und der Aufbewahrungsdauer | | | | | |
|---|---|---|---|---|---|
| Aufbewahrung | | gemessene Titer der HIV-Infektiosität (ausgedrückt in TCID-50-Einheiten pro ml) | | | |
| Temp. (°C) | Dauer (Tage) | Ansätze | | | |
| | | (d) Kontrolle kein 32P | (a) 1 32P auf 30 Genomkopien | (b) 1 32P auf 3 Genomkopien | (c) 1 32P auf 1 Genomkopie |
| +4 | 0 | $2{,}33 \times 10^5$ | $1{,}46 \times 10^5$ | $2{,}33 \times 10^5$ | $1{,}46 \times 10^5$ |
| | 7 | $1{,}46 \times 10^5$ | $4{,}86 \times 10^5$ | $1{,}62 \times 10^4$ | $1{,}80 \times 10^3$ |
| | 14 | $1{,}80 \times 10^3$ | $1{,}80 \times 10^3$ | $2{,}00 \times 10^2$ | $2{,}00 \times 10^2$ |
| | 28 | $10^2$ | $2 \times 10^2$ | $<6 \times 10$ | $<6 \times 10$ |

Tabelle 2 (fortgesetzt)

| Abnahme der HIV-Infektiosität in Abhängigkeit von, der 32-P-Konzentration, der Aufbewahrungstemperatur und der Aufbewahrungsdauer | | | | | |
|---|---|---|---|---|---|
| Aufbewahrung | | gemessene Titer der HIV-Infektiosität (ausgedrückt in TCID-50-Einheiten pro ml) | | | |
| Temp. (°C) | Dauer (Tage) | Ansätze | | | |
| | | (d) Kontrolle kein 32P | (a) 1 32P auf 30 Genomkopien | (b) 1 32P auf 3 Genomkopien | (c) 1 32P auf 1 Genomkopie |
| -70 | 0 | $1,46 \times 10^5$ | $2,33 \times 10^5$ | $1,46 \times 10^5$ | $1,46 \times 10^5$ |
| | 7 | $1,46 \times 10^5$ | $2,80 \times 10^4$ | $9,30 \times 10^3$ | $5,40 \times 10^3$ |
| | 14 | $1,46 \times 10^5$ | $2,80 \times 10^4$ | $1,62.10^3$ | $3,10 \times 10^3$ |
| | 28 | $1,46 \times 10^5$ | $1,62 \times 10^2$ | $6 \times 10$ | $<6 \times 10$ |
| -170 | 0 | $2,33 \times 10^5$ | $2,33 \times 10^5$ | $1,46 \times 10^5$ | $1,46 \times 10^5$ |
| | 7 | $1,46 \times 10^5$ | $4,86 \times 10^4$ | $2,80 \times 10^4$ | $5,40 \times 10^3$ |
| | 14 | $2,33 \times 10^5$ | $2,80 \times 10^4$ | $9,30 \times 10^3$ | $1,80 \times 10^3$ |
| | 28 | $1,46 \times 10^5$ | $1,62 \times 10^2$ | $<6 \times 10$ | $<6 \times 10$ |

Die vorstehenden Ergebnisse bestätigen eine hochwirksame und irreversible Inaktivierung von HIV, weil durch den radioaktiven Zerfall die RNA-Stränge unterbrochen werden und weil es keine Möglichkeit zur natürlichen Reparatur oder Regenerierung gibt. Die auf diese Weise gestörte Virusstruktur könnte unter den Bedingungen einer außerordentlich hohen Multiplizität der Infektion wieder regeneriert werden, die eine Komplettierung der defekten Virusstruktur ermöglicht. Diese Möglichkeit ist jedoch vernachläßigbar, weil der HIV-Titer im Blut nur sehr gering ist (ungefähr 100 Viruspartikel pro ml).

Davon ausgehend, wird mit der vorliegenden Erfindung vorgeschlagen, radioaktiven Phosphor $^{32}_{15}$P in die RNA-Struktur des aktiven Virus während dessen Synthese einzubauen, und während der Phase des radioaktiven Zerfalls von $^{32}_{15}$P zu $^{32}_{16}$S die Präparate unter Bedingungen zu halten, die eine Regenerierung der Bruchstellen ausschließen. Am einfachsten kann dies dadurch geschehen, daß die Präparate nach dem Einbau von radioaktivem Phosphor $^{32}_{15}$P für eine Zeitspanne von wenigstens zwei Halbwertszeiten (im vorliegenden Falle etwa 28 Tage) bei einer Temperatur von wenigstens minus 70°C, vorzugsweise bei einer Temperatur von minus 170°C gehalten werden. Auf diese Weise kann die RNA-Struktur des Virus von innen heraus zerstört werden, ohne daß gleichzeitig auch die antigenen Eigenschaften des Virus zerstört werden, die hauptsächlich auf den Proteinen in und an der Virushülle beruhen. Entsprechend dem erfindungsgemäßen Verfahren zur Herstellung einer Vakzine zur Prophylaxe gegen eine HIV-Infektion und/oder Therapie von AIDS wird in vitro radioaktiver Phosphor $^{32}_{15}$P in die RNA-Struktur von HIV eingebaut. Die so erhaltenen Proben werden für eine längere Zeitspanne bei einer Temperatur von wenigstens minus 70°C oder tiefer, vorzugsweise bei der Temperatur des flüssigen Stickstoffs (minus 170°C) aufbewahrt, um den Wirkstoff für eine wirksame und gefahrlos anwendbare und immunogene Vakzine zu erhalten. Diese Aufbewahrungsdauer bei tiefer Temperatur soll wenigstens zwei Halbwertszeiten von $^{32}_{15}$P, also wenigstens ca. 28 Tage betragen. Vorzugsweise soll die Dauer dieer Tief-Temperatur-Aufbewahrung mehr als zwei Monate betragen. Besonders bevorzugt soll die Dauer dieser Tief-Temperatur-Aufbewahrung nicht weniger als 4 Monate und nicht länger als 6 Monate betragen. Im Hinblick auf die Halbwertszeit von $^{32}_{15}$P von 14,22 Tagen gewährleistet eine Dauer der Tief-Temperatur-Aufbewahrung von wenigstens 4 Monaten seit Einbau der radioaktiven $^{32}_{15}$P-Isotope, daß die so aufgenommene Eigen-Radioaktivität auf einen vernachlässigbaren Wert abgesunken ist.

Ein besonderer Vorteil dieses erfindungsgemäßen Verfahrens, und ein deutlicher Unterschied zu anderen bekannten Vorschlägen zur Bereitstellung einer Vakzine mit inaktiviertem Gesamtvirus besteht darin, daß erfindungsgemäß die Virusstruktur von innen heraus zerstört werden kann, wodurch die für die Vermehrung wichtige Genstruktur beseitigt wird, während die für die Aktivierung und Provozierung der Immunreaktion wichtige Antigenität erhalten bleibt.

Die nachstehenden Berechnungen dienen dazu, das optimale Verhältnis von radioaktivem Phosphor zu nicht radioaktivem Phosphor in dem Kulturmedium sowie die optimale Dauer der Aufbewahrung bei tiefer Temperatur abzuschätzen und theoretisch abzusichern. Es gilt die Randbedingungen für die verschiedenen Maßnahmen zu ermitteln, um eine wirksame und gefahrlos anwendbare Vakzine bereitzustellen.

Die Geschwindigkeit des radioaktiven Zerfalls entspricht derjenigen einer monomolekularen Reaktion; das

7

heißt, die je Zeiteinheit zerfallende Menge eines radioaktiven Stoffes ist in jedem Augenblick der noch vorhandenen Menge proportional. Der Proportionalitätsfaktor ist die Zerfallskonstante. Diejenige Zeitspanne, in der gerade die Hälfte einer vorhandenen Menge radioaktiver Substanz umgewandelt oder zerfallen ist, wird als Halbwertszeit bezeichnet, Es gilt

$$N = No \times 2^{-t/T} \tag{1}$$

wobei :

T =    die Halbwertszeit;
t =    die jeweils aktuelle Zeitspanne;
No =    die anfänglich vorhandene Anzahl radioaktiver Isotope;
N =    diejenige Anzahl der Isotope, die im Verlauf der Zeitspanne "t" noch nicht zerfallen ist.

Aus Gleichung (1) läßt sich die Wahrscheinlichkeit "a" errechnen, daß irgendein Isotop innerhalb der Zeitspanne "t" unverändert zurückbleibt:

$$a = 2^{-t/T} \tag{2}$$

Für die weiteren Betrachtungen werden nachstehende Größen eingeführt:

$C_p$ =    Konzentration an nicht-radioaktivem Phosphor im Kulturmedium;
$C_r$ =    Konzentration an radioaktivem Phosphor ($^{32}_{15}$P) im Kulturmedium;
Mv =    die Anzahl der Phosphoratome in der einsträngigen RNA-Struktur des HIV-Genomes (angenähert 9.500).

Hieraus errechnet sich der Anteil (n) der radioaktiven Phosphoratome, die bei der Reproduktion der Virus-RNA pro RNA-Strang eingebaut werden zu:

$$n = Cr \times Mv/Cp \tag{3}$$

Da jedes $^{32}_{15}$P-Isotop statistisch gesehen unabhängig von jedem anderen P-Isotop zerfällt, ergibt sich die Wahrscheinlichkeit, daß innerhalb der Zeitspanne "t" überhaupt kein $^{32}_{15}$P-Atom innerhalb des RNA-Moleküles zerfällt, bzw. die Wahrscheinlichkeit "A", daß die HIV-RNA-Struktur reproduzierbar bleibt zu

$$A = 2^{-tn/T} = (2^{-t/T})^{Cr \times Mv/Cp} \tag{4}$$

Es kann nunmehr ein Schwellenwert "$A_{max}$" gewählt werden, welcher die maximal akzeptierbare Wahrscheinlichkeit der Aktivität oder Virulenz der Viruspartikel ausdrückt. Dieser Schwellenwert kann auf der Basis nachstehender Überlegungen ausgewählt werden. Hierbei wird davon ausgegangen, daß es erforderlich ist, eine Anzahl von "v" Viruspartikel in den Organismus einzubringen, um eine erfolgreiche Immunisierung zu erzielen. Sofern der Schwellenwert $A_{max}$ zu groß gewählt wird, dann verbleiben - entsprechend dem Gesetz der großen Zahl - ein oder mehrere Viruspartikel virulent. Sofern z.B. v = 100.000 gewählt wird und $A_{max}$ = $10^4$ (das heißt 0,01 %) angenommen wird, dann bleiben aus der Gesamtzahl von 100.000 in den Organismus eingebrachten Viruspartikel 10 Viruspartikel virulent. Eine solche Situation ist nicht akzeptabel, da bereits von einem einzigen aktiven Viruspartikel eine Infektion ausgehen kann.

Entsprechend einem anderen Beispiel wird von der Anzahl v = $10^5$ und von dem Schwellenwert $A_{max}$ = $10^{-7}$ ausgegangen; In diesem Falle ist die Wahrscheinlichkeit, daß ein einziges Viruspartikel virulent bleibt, gleich 1 %. Da die Werte für Mv und v vergleichsweise große Zahlen sind, und das Gesetz der großen Zahl auch in diesem Falle gilt, ist eine Abweichung von 1 % von diesen großen Zahlen vernachlässigbar. Dies bedeutet in diesem Falle, daß es als unmöglich angesehen wird, daß von einem einzigen verbleibenden aktiven Viruspartikel eine Infektionsgefahr ausgehen kann.

Auf der Basis der vorausgegangenen Beispiele wird der Schwellenwert $A_{max}$ gewählt zu

$$A_{max} = 0{,}01v \tag{5}$$

Für ein anderes Beispiel könnte theoretisch $A_{max}$ auch so vernachlässigbar klein gewählt werden, daß selbst dann, wenn die gesamte Erdbevölkerung immunisiert werden sollte, keine einzige Person ein virulentes Viruspartikel erhält. In diesem Falle müßte der Schwellenwert $A_{max}$ angesetzt werden zu

$$A_{max} = v \times 10^{-10} \tag{6}$$

Wird der Schwellenwert $A_{max}$ anstelle der Wahrscheinlichkeit "A" in Gleichung (4) eingesetzt, so kann die Zeitspanne "t" errechnet werden, die erforderlich ist, für die Aufbewahrung des Präparates bei tiefer Temperatur, um eine gefahrlos anwendbare Vakzine zu erhalten. Aus der zweiten Alternative der Gleichung (4) ist auch ersichtlich, daß für verschiedene Systeme mit unterschiedlichen Werten von "n" und dem Quotient "Cr/Cp" unterschiedliche Werte für diese notwendige Zeitspanne "t" erhalten werden.

$$t = -T/(n\ \lg 2) \times \lg A_{max} = -T/\lg 2 \times Cp/(Cr\ Mv) \times \lg A_{max} \tag{7}$$

oder

$$t = -3{,}32\ T/n \times \lg A_{max} = -3{,}32\ T\ Cp/(Cr\ Mv) \times \lg A_{max} \tag{8}$$

Fall 1:

Hier wird von den Werten $v = 100.000$ und $A_{max} = 10^{-7}$ ausgegangen.
In diesem Falle gilt

$$t = 3{,}32 \times 7 \times T/n = 23T/n$$

Bekanntlich beträgt T für $^{32}_{15}P$ 14,22 Tage. Sofern $n = 1$ annehmen soll, dann muß die $^{32}_{15}P$-haltige RNA-Struktur 330 Tage lang bei tiefer Temperatur gehalten werden; dies ist nahezu ein Jahr.
Sofern andererseits gelten soll, $n = 10$, dann muß die $^{32}_{15}P$-haltige RNA-Struktur 33 Tage lang bei tiefer Temperatur gehalten werden, das ist etwa ein Monat lang.

Fall 2:

Hier wird von dem Ziel ausgegangen, eine solch gefahrlos anwendbare Vakzine zu schaffen, daß selbst bei einer Immunisierung der gesamten Weltbevölkerung kein virulentes Viruspartikel injiziert wird. Sofern wiederum von der Anzahl für eine wirksame Immunisierung der zu injizierenden, inaktiven Viruspartikel von $v = 100.000$ ausgegangen wird, dann muß das $^{32}_{15}P$-haltige Ausgangsmaterial bei kalter Temperatur aufbewahrt werden

im Falle von        $n = 1$ für 1,9 Jahre
im Falle von        $n = 10$ für 71 Tage.

Sofern die Anzahl von 100.000 inaktivierter Viruspartikel als nicht ausreichend angesehen wird, sondern $v = 10^7$ inaktivierte Viruspartikel für eine erfolgreiche Immunisierung angestrebt werden, dann gilt für die Aufbewahrung des Ausgangsmaterials bei tiefen Temperaturen:

im Falle von        $n = 1$ für 2,2 Jahre
im Falle von        $n = 10$ für 2 Monate und 20 Tage
im Falle von        $n = 20$ für 1 Monat und 10 Tage.

Im Falle obiger Überlegungen ist eine gewisse Unsicherheit bei der Bestimmung von "Cr" und "Cp" nicht berücksichtigt worden. Diese Unsicherheit kann jedoch zusätzlich mit dem Meßfehler $\delta$ % berücksichtigt werden. So-

fern im Falle einer Reproduktion (n = 1) eine Probe keinerlei radioaktiven Phosphor $^{32}_{15}$ P aufnimmt, so bleiben $\delta$ % der Viruspartikel weiterhin infektiös, unabhängig von der Dauer der Aufbewahrung bei tiefer Temperatur. In diesem Falle kann der Fall von n = 1 nicht als sehr gefährlich angesehen werden.

Der kleinste, noch hinnehmbare Wert für "n" scheint zu sein

$$n = 1 + 2\delta \qquad (9)$$

Sofern beispielsweise $\delta$ = 5 % angenommen wird, dann errechnet sich dieser kleinste akzeptable Wert von "n" zu 1,1.

Aus der nachstehenden Betrachtung ergibt sich, daß durchaus eine Korrelation zwischen dem eingangs aufgeführten Beispiel mit obigen Überlegungen besteht.

Das für die vorliegenden Arbeiten eingesetzte radioaktive Phosphat weist eine Aktivität von 200 MBq (Mega Becquerel) auf; das entspricht einer Aktivität von 2 x 10$^8$ Bq pro Ansatz von 200 µl.

1 Mol radioaktive Phosphorsäure, bei der sämtliche P-Atome aus radioaktivem $^{32}_{15}$ P bestehen, weist eine molare Aktivität von 1,85 x 10$^{17}$ Bq auf. Hieraus läßt sich errechnen, daß für die Versuche 1,1 x 10$^{-9}$ Mol radioaktives Phosphat zur Verfügung gestanden haben. Dieser Gesamtansatz wurde in nachfolgende Mengen unterteilt:

(a) 25 ml Medium werden mit 2,8 x 10$^{-11}$ Mol radioaktivem Phosphat versetzt; dies liefert ein Medium mit einer Radioaktivität von 5,2 µCi/ml;

(b) 25 ml Medium werden mit 2,8 x 10$^{-10}$ Mol radioaktivem Phosphat versetzt; dies liefert ein Medium mit einer Radioaktivität von 46,8 µCi/ml;

(c) 25 ml Medium werden mit 8,0 x 10$^{-10}$ Mol radioaktivem Phosphat versetzt; dies liefert ein Medium mit einer Radioaktivität von 156,0 µCi/ml.

Wie bereits gesagt, beträgt die Halbwertszeit von $^{32}_{15}$ P 14,22 Tage, das sind angenähert 20.477 min.

Aus der vorhandenen Mol-Menge an $^{32}_{15}$ P pro Ansatz zerfällt jeweils die Hälfte pro Halbwertszeit. Hieraus kann erechnet werden, welcher Anteil der Mol-Menge pro Minute zerfällt:

(a) 1,4 x 10$^{-11}$ : 2,048 x 10$^4$ = 6,83 x 10$^{-16}$ Mol
(b) 1,4 x 10$^{-10}$ : 2,048 x 10$^4$ = 6,83 x 10$^{-15}$ Mol
(c) 4,0 x 10$^{-10}$ : 2,048 x 10$^4$ = 1,95 x 10$^{-14}$ Mol

Durch Multiplizierung dieser Zahlen mit der Avogadro-schen Zahl (6,0 x 10$^{23}$) kann aus vorstehenden Mol-mengen die Anzahl der $^{32}_{15}$ P-Isotope berechnet werden, die in jedem Ansatz pro Minute zerfällt:

(a) 40,9 x 10$^7$
(b) 40,9 x 10$^8$
(c) 11,7 x 10$^9$

Aus jedem Ansatz "a", "b" und "c" werden jeweils die drei Fraktionen (je 15 µl) mit der höchsten Radioaktivität vereinigt. Die so erhaltenen Anteile weisen nachstehende molare Mengen an radioaktivem Phosphor $^{32}_{15}$ P auf.

(a) 67.460 x 2,8$^{-11}$ : 40,98 x 10$^7$ = 4,6 x 10$^{-15}$M
(b) 389.349 x 2,8$^{-10}$ : 40,98 x 10$^8$ = 2,7 x 10$^{-14}$M
(c) 785.925 x 8,0$^{-10}$ : 11,70 x 10$^9$ = 5,4 x 10$^{-14}$M

Diese molaren Anteile ergeben die folgenden Werte für die Anzahl der $^{32}_{15}$ P-Isotope:

(a) 2,70 x 10$^9$
(b) 1,56 x 10$^{10}$
(c) 3,18 x 10$^{10}$

Damit enthalten 600 µl Lösung der Fraktionen mit der höchsten Radioaktivität aus jedem Ansatz "a", "b" und "c" jeweils

(a) $3,60 \times 10^{10}$ Isotope $^{32}_{15}$P
(b) $2,10 \times 10^{11}$ Isotope $^{32}_{15}$P
(c) $4,20 \times 10^{11}$ Isotope $^{32}_{15}$P

Wie oben anläßlich der Beschreibung des Versuches ausgeführt, enthalten 600 µl Lösung der Fraktionen mit der höchsten Radioaktivität angenähert 7.200 ng p24-Antigen; das liefert $7.200 \times 10^9/(2,4 \times 10^4 \times 1,7 \times 10^{-24}) = 1,76 \times 10^{14}$ Moleküle p24 auf 600 µl Lösung. Da 1 HIV-Partikel 1.024 p24 Moleküle aufweist, läßt sich aus dieser Zahl errechnen, daß 600 µl Lösung angenähert $1,72 \times 10^{11}$ Viruspartikel enthalten.

Bekanntlich enthält jedes HIV-Partikel zwei Genomkopien, von den jede eine einsträngige RNA-Struktur mit angenähert 9.500 Nukleotiden aufweist. Die Anzahl der Phosphat-Di-Estergruppen entspricht der Anzahl der Nukleotide. Damit enthält jedes Viruspartikel etwa $1,9 \times 10^4$ Phosphatgruppen. Damit enthalten alle Viruspartikel in 600 µl Lösung der Fraktionen mit der höchsten Radioaktivität $3,27 \times 10^{15}$ Phosphatgruppen. Damit kann aus den oben ermittelten Anzahlen der $^{32}_{15}$P-Isotope in je 600 µl Lösung aus den verschiedenen Ansätzen (a) bis (c) wiederum auf die Anzahl radioaktiver $^{32}_{15}$P-Isotope in diesen Ansätzen geschlossen werden. In den Ansätzen "a", "b" und "c" kommen auf 1 radioaktives $^{32}_{15}$P-Isotop die nachstehenden Di-Phosphat-Estergruppen:

| | | $^{32}_{15}$P-Konzentration µCi/ml |
|---|---|---|
| a) | $3,27 \times 10^{15} : 3,6 \times 10^{10} = 90.833$ | 5,2 |
| b) | $3,27 \times 10^{15} : 2,1 \times 10^{11} = 15.571$ | 46,8 |
| c) | $3,27 \times 10^{15} : 4,2 \times 10^{11} = 7.785$ | 156,0 |

Wie dargelegt, korrespondiert dies mit der Radioaktivität des jeweiligen Kulturmediums "a", "b" und "c". Damit ist eine ausreichende Übereinstimmung zwischen den Versuchsergebnissen und den mehr theoretischen Berechnungen dargelegt.

Aus obigen Ergebnissen kann auch geschlossen werden, daß unter den gewählten Bedingungen ein Verhältnis von 1 Teil radioaktives Phosphorisotop $^{32}_{15}$P auf 10.000 Teile nicht-radioaktives Phosphorisotope $^{31}_{15}$P im Kulturmedium zum Einbau von etwa einem $^{32}_{15}$P-Isotop in ein einzelnes Genom von HIV führt. Entsprechend führt unter den gewählten Bedingungen ein Verhältnis 4 Teile $^{32}_{15}$P auf 10.000 Teile $^{32}_{15}$P im Kulturmedium zum Einbau von etwa vier $^{32}_{15}$P-Isotope in ein einzelnes HIV-Genom.

In einem weiteren Versuch wurde geprüft, ob die erzielbare antigene Wirkung abhängig ist von der aufgenommenen Menge an radioaktivem Phosphor. Diese Ergebnisse sollten Hinweise darüber liefern, ob die Aufnahme und der Zerfall von $^{32}_{15}$P nicht nur die RNA-Struktur der Viruspartikel unterbricht, sondern gegebenenfalls auch die Proteinstruktur an der Virushülle beeinträchtigt, welche die Antigenität hervorruft.

Anteile der vorstehend genannten Ansätze (a) bis (d) werden 2 Monate lang bei -70°C gehalten, um in den aktiven Präparaten (a) bis (c) die überwiegende Anzahl an $^{32}_{15}$P-Isotopen zerfallen zu lassen. Die Kontrollprobe (d) wird unter gleichen Bedingungen gehalten. Die so erhaltenen, aktives Virus enthaltenden Präparate werden mit Freund'schen Adjuvans vermischt. In drei parallelen Versuchsreihen wird je eine Maus von 15 bis 18 g mit diesen Präparaten immunisiert. Hierzu werden jeder Maus je 15 µg-Geamt-Antigen subcutan injiziert. Die Blutsera werden am 14. Tag nach der Immunisierung gewonnen. Die Blutsera werden 30 min lang bei 56°C gehalten und daraufhin zweifach seriell im Verhältnis 1 : 10 bzw. 1 : 20 bzw. 1 : 40 bis 1 : 1280 mit einem Zellwuchsmedium verdünnt. Die so erhaltenen seriellen Verdünnungen werden mit je 100 bis 5000 Syncythia bildenden Einheiten (SFU von syncytia forming units) von aktiven gezüchteten HIV-Partikeln (Stamm RF von HIV-1) versetzt und 0,5 bis 1,0 Stunden lang bei 37°C praeincubiert, damit die möglicherweise gebildeten Antikörper auf die aktiven Viruspartikel einwirken können. Daraufhin wird jedes Blutserum/Virus-Gemisch zu einer CEM-SS Suspension hinzugefügt, die sich in den Näpfchen einer Platte befinden; jeder Versuch wird zweifach durchgeführt. Die Präperate werden 4 Tage lang unter Wachstumsbedingungen (37°C, 5 % Kohlendioxid) gehalten. Daraufhin wird die mittlere ID$_{50}$ bestimmt. Die Anzahl der Viren wird am 5. Tag auf optischem Wege mit Hilfe des ELISA-Tests zum p24-Antigen bestimmt. Diejenige serielle Serumverdünnung, welche die Virusinfektion zu 50 % zu inhibieren vermag, wird als "50 % der Viren neutralisierende Verdünnung" bezeichnet. Je größer die jeweilige Verdünnung ist, desto größer ist die Virusneutralisierende Aktivität des jeweiligen Serums. Die Ergebnisse sind in der nachfolgenden Tabelle 3 dargestellt:

Tabelle 3

| 50 % der Viren neutralisierender Titer der geprüften Sera | |
|---|---|
| Anzahl der $^{32}_{15}$ P-Isotope pro HIV-Genom | 50 % der Viren neutralisierende Verdünnung des ursprünglichen Hyperimmunserums |
| (a)  0,03 | 1 : 80 |
| (b)  0,30 | 1 : 160 |
| (c)  1,00 | 1 : 112 |
| (d)  0,00 (Kontrollprobe) | 1 : 112 |
| keine Antigen-Praeimmunisierung (neg. Kontrollprobe) | <1 : 10 |

Aus diesen Ergebnissen ist zu schließen, daß der unterschiedliche $^{32}_{15}$ P-Gehalt der HIV-Genome praktisch keine Auswirkungen auf die antigenen Eigenschaften hat. Es ist praktisch keine Korrelation zwischen der Menge an aufgenommenem $^{32}_{15}$ P pro HIV-Genom und der die Synzytien-Bildung inhibierenden Aktivität der Hyperimmunseren zu erkennen. Daraus muß geschlossen werden, daß trotz der "von innen heraus" erfolgenden Inaktivierung der Viruspartikel durch Aufnahme von $^{32}_{15}$ P deren antigene Struktur erhalten geblieben ist, so daß diese inaktivierten HIV-Partikel eine spezifische Antikörperantwort provozieren können. Damit können diese inaktivierten HIV-Partikel als Wirkstoff einer Vakzine zur Prophylaxe gegen eine HIV-Infektion und/oder zur Therapie von AIDS dienen.

Ähnliche Ergebnisse werden für die Impfung gegen andere Retroviren erwartet, wenn zur Herstellung der erforderlichen Vakzine ein angepaßtes Verfahren gemäß der vorliegenden Erfingung angewandt wird.

**Patentansprüche**

1.  Vakzine zur Prophylaxe gegen eine HIV-Infektion und/ oder zur Therapie von AIDS,
    mit einem Gehalt an inaktivierten HIV-Partikeln,
    dadurch gekennzeichnet, daß
    die inaktivierten HIV-Partikel erhältlich sind durch

    -   Kultivierung von HIV in einem Kulturmedium, das als Phosphorquelle das radioaktive Phosphorisotop $^{32}_{15}$ P enthält;
    -   Isolierung und Abtrennung der kultivierten HIV-Partikel aus dem Kulturmedium unter Bedingungen, die wenigstens 50 % oder mehr des viralen gp120-Antigens erhalten; und
    -   Aufbewahrung der so gebildeten und isolierten HIV-Partikel, deren RNA wenigstens eine Phosphorsäure-Di-Estergruppe enthält, deren Phosphorspezies aus $^{32}_{15}$ P besteht für einen Zeitraum von mehr als zwei Monaten bei einer Temperatur von minus 70° oder unterhalb minus 70°C.

2.  Vakzine nach Anspruch 1,
    dadurch gekennzeichnet, daß
    die Kultivierung von HIV unter solchen Bedingungen erfolgt ist, unter denen jedes einzelne Genom der HIV-Partikel wenigstens 1 bis 4 radioaktive $^{32}_{15}$ P-Isotope aufgenommen hat.

3.  Vakzine nach Anspruch 1 oder 2,
    dadurch gekennzeichnet, daß
    das inaktivierte HIV-Partikel ein HIV-Gesamt-Virion ist.

4.  Verfahren zur Herstellung einer Vakzine zur Prophylaxe gegen eine HIV-Infektion und/oder zur Therapie von AIDS,
    gekennzeichnet durch die Verfahrensschritte:

    -   aktive HIV-Partikel werden in einem Kulturmedium vermehrt, das als Phosphorquelle das radioaktive Phosphorisotop $^{32}_{15}$ P enthält;
    -   die so gebildeten HIV-Partikel werden aus dem Kulturmedium unter solchen Bedingungen abgetrennt und isoliert, die wenigstens 50 % oder mehr des viralen gpl20-Antigens erhalten; und
    -   die so isolierten HIV-Partikel, deren RNA wenigstens eine Phosphorsäure-Di-Estergruppe enthält, deren Phosphorspezies aus $^{32}_{15}$ P besteht, werden für einen Zeitraum von mehr als zwei Monaten bei einer Temperatur von minus 70°C oder unterhalb minus 70°C aufbewahrt; und

- daraufhin werden diese HIV-Partikel zu einer applizierbaren Vakzine konfektioniert.

5. Verfahren nach Anspruch 4,
   dadurch gekennzeichnet, daß
   die zur Konfektionierung der applizierbaren Vakzine eingesetzten inaktivierten HIV-Partikel HIV-Gesamt-Virions sind.

6. Verfahren nach Anspruch 4,
   dadurch gekennzeichnet, daß
   die Vermehrung der HIV-Partikel in einem Kulturmedium durchgeführt wird, das durch Zugabe von $^{32}_{15}$P wenigstens eine Radioaktivität von 5 µCi (Mikrocurie) pro 1 ml Kulturmedium aufweist.

7. Verfahren nach Anspruch 4,
   dadurch gekennzeichnet, daß
   die Vermehrung der HIV-Partikel in einem Kulturmedium durchgeführt wird, das aufgrund der Zugabe von $^{32}_{15}$P eine Radioaktivität von 5 bis 200 µCi pro 1 ml Kulturmedium aufweist.

8. Verfahren nach Anspruch 4,
   dadurch gekennzeichnet, daß
   die Vermehrung der HIV-Partikel in einem Kulturmedium durchgeführt wird, das auf 300.000 nicht-radioaktive P-Isotope wenigstens 1 radioaktives $^{32}_{15}$P-Isotop enthält.

9. Verfahren nach Anspruch 4,
   dadurch gekennzeichnet, daß
   die Vermehrung der HIV-Partikel in einem Kulturmedium durchgeführt wird, das auf 30.000 nicht-radioaktive P-Isotope wenigstens 1 radioaktives $^{32}_{15}$P-Isotop aufweist.

10. Verfahren nach Anspruch 4,
    dadurch gekennzeichnet, daß
    die Vermehrung der HIV-Partikel in einem Kulturmedium durchgeführt wird, das auf 10.000 nicht-radioaktive P-Isotope wenigstens 1 radioaktives $^{32}_{15}$P-Isotop aufweist.

11. Verfahren nach einem der Ansprüche 4 bis 10,
    dadurch gekennzeichnet, daß
    die so gebildeten und isolierten HIV-Partikel, deren RNA wenigstens einige Phosphorsäure-Di-Estergruppen enthält, deren Phosphorspezies aus $^{32}_{15}$P besteht, bei einer Temperatur des flüssigen Stickstoffs (minus 170°C) aufbewahrt werden.

12. Verfahren nach Anspruch 11,
    dadurch gekennzeichnet, daß
    die HIV-Partikel in einem Kulturmedium vermehrt werden, das auf 10.000 nicht-radioaktive P-Isotope wenigstens 4 radioaktive $^{32}_{15}$P-Isotope enthält; und
    die so gebildeten und isolierten HIV-Partikel nicht weniger als 4 Monate lang bei der Temperatur des flüssigen Stickstoffs aufbewahrt werden.

13. Verfahren nach Anspruch 11,
    dadurch gekennzeichnet, daß
    die HIV-Partikel in einem Kulturmedium vermehrt werden, das auf 10.000 nicht-radioaktive P-Isotope wenigstens 3 radioaktive $^{32}_{15}$P-Isotope enthält; und
    die so gebildeten und isolierten HIV-Partikel nicht mehr als 6 Monate lang bei der Temperatur des flüssigen Stickstoffs aufbewahrt werden.

14. Verfahren nach Anspruch 4,
    dadurch gekennzeichnet, daß
    die HIV-Partikel in einem Kulturmedium unter solchen Bedingungen vermehrt werden, daß jedes einzelne HIV-Genom wenigstens 1 bis 4 radioaktive $^{32}_{15}$P-Isotope aufnimmt.

**15.** Verfahren nach Anspruch 14,
underline: dadurch gekennzeichnet, daß
die durch Aufnahme von $^{32}_{15}$ P inaktivierten HIV-Partikel 4 bis 6 Monate lang bei der Temperatur des flüssigen Stickstoffs aufbewahrt werden.

**16.** Verfahren nach einem der Ansprüche 4 bis 15,
dadurch gekennzeichnet, daß
solche HIV-Partikel vermehrt werden, die wenigstens einen gewissen Polymorphismus in ihrer Genomstruktur aufweisen.


**Claims**

**1.** A vaccine
for prophylaxis against an infection caused by HIV and/or for therapy of AIDS,
comprising a content of inactivated HIV particles
characterized in that
said inactivated HIV particles are obtainable by a process comprising the following steps:

- cultivating HIV within a growth medium comprising a source of phosphorus which contains a radioactive phosphorus isotope $^{32}_{15}$ P;
- harvesting and separating the thus cultivated HIV particles from said growth medium under conditions preserving at least 50 % or more of viral gp 120 antigen; and
- maintaining the thus obtained HIV particles comprising a RNA having at least one phosphoric acid di-ester group, the phosphorus thereof comprises radioactive $^{32}_{15}$ P for a period of more than two months at a temperature of minus 70°C or at a temperature lower than minus 70°C.

**2.** The vaccine according to claim 1,
characterized in that
effecting said cultivating of HIV under those conditions providing that each single genome of the HIV particles has incorporated at least 1 to 4 radioactive $^{32}_{15}$ P isotope(s).

**3.** The vaccine according to claim 1 or 2,
characterized in that
said inactivated HIV particle comprises a whole HIV virion.

**4.** A method of preparing a vaccine for prophylaxis against an infection caused by HIV and/or for therapy of AIDS,
characterized by the following method steps:

- cultivating active HIV particles within a growth medium comprising a source of phosphorus which contains a radioactive phosphorus isotope $^{32}_{15}$ P;
- harvesting and separating the thus cultivated HIV particles from said growth medium under conditions preserving at least 50 % or more of viral gp 120 antigen; and
- maintaining the thus obtained HIV particles comprising a RNA having at least one phosphoric acid di-ester group the phosphorus thereof comprises radioactive $^{32}_{15}$ P for a period of more than two months at a temperature of minus 70°C or lower; and
- formulating these HIV particles to a vaccine ready for application.

**5.** The method according to claim 4,
characterized in that
said HIV particles used for formulating said applicable vaccine comprise whole HIV virions.

**6.** The method according to claim 4,
characterized in that
cultivating HIV particles within a growth medium comprising per 1 ml growth medium a radioactivity of at least 5 μCi (microCurie) due to an addition of a sufficient amount of radioactive $^{32}_{15}$ P.

**7.** The method according to claim 4,

characterized in that

cultivating HIV particles within a growth medium comprising per 1 ml growth medium a radioactivity of 5 to 200 μCi (microCurie) due to an addition of a sufficient amount of radioactive $^{32}_{15}$ P.

8. The method according to claim 4,
   characterized in that
   cultivating HIV particles within a growth medium comprising at least one radioactive $^{32}_{15}$ P isotope per 300,000 non-radioactive P isotopes.

9. The method according to claim 4,
   characterized in that
   cultivating HIV particles within a growth medium comprising at least one radioactive $^{32}_{15}$ P isotope per 30,000 non-radioactive P isotopes.

10. The method according to claim 4,
    characterized in that
    cultivating HIV particles within a growth medium comprising at least one radioactive $^{32}_{15}$ P isotope per 10,000 non-radioactive P isotopes.

11. The method according to anyone of the claims 4 to 10,
    characterized in hat
    maintaining the thus formed and separated HIV particles comprising a RNA having at least one phosphoric acid di-ester group the phosphorus moiety thereof consists of radioactive $^{32}_{15}$ P at the temperature of liquid nitrogen (minus 170°C).

12. The method according to claim 11,
    characterized in that
    cultivating HIV particles within a growth medium comprising at least four radioactive $^{32}_{15}$ P isotopes per 10,000 non-radioactive P isotopes; and
    maintaining the thus formed and separated HIV particles for a period of non less than 4 months a the temperature of liquid nitrogen.

13. The method according to claim 11,
    characterized in that
    cultivating HIV particles within a growth medium comprising at least three radioactive $^{32}_{15}$ P isotopes per 10,000 non-radioactive P isotopes; and
    maintaining the thus formed and separated HIV particles for a period of not more than 6 months at the temperature of liquid nitrogen.

14. The method according to claim 4,
    characterized in that
    cultivating HIV particles within a growth medium under those conditions providing that each single HIV genome incorporates at least 1 to 4 radioactive $^{32}_{15}$ P isotopes.

15. The method according to claim 14,
    characterized in that
    maintaining the HIV particles inactivated by said incorporation of $^{32}_{15}$ P for a period of 4 to 6 months at the temperature of liquid nitrogen.

16. The method according to anyone of the claims 4 to 15,
    characterized in that
    cultivating those HIV particles comprising at least a certain degree of polymorphism of their genome structure.

**Revendications**

1. Vaccin pour la prophylaxie contre une infection causée par le virus HIV et/ou pour la thérapeutique du S.I.D.A., comprenant une teneur en particules inactivées du virus HIV

caractérisé en ce que,
les particules inactivées du virus HIV sont obtenues

- en cultivant HIV dans un milieu de culture ayant une source de phosphore qui comprend l'isotope radioactif de phosphore $^{32}_{15}$P ;
- en isolant et séparant les particules HIV cultivées dans le milieu de culture, sous des conditions préservant au moins 50 % ou plus de l'antigène viral gp 120; et
- en stockant les particules HIV ainsi formées et isolées dont le génome RNA comprend au moins un groupe acide phosphorique diester dont le phosphore comprend $^{32}_{15}$P, pendant une période de plus de deux mois à une température égale ou inférieure à moins 70°C.

2. Vaccin suivant la revendication 1,
caractérisé en ce que
HIV a été cultivé sous des conditions assurant que tout génome individuel des particules HIV a absorbé au moins 1 à 4 isotopes radioactifs $^{32}_{15}$P.

3. Vaccin suivant la revendication 1 ou 2,
caractérisé en ce que
la particule HIV inactivée est un virion HIV complet.

4. Procédé de fabrication d'un vaccin pour la prophylaxie contre une infection causée par le virus HIV et/ou pour la thérapeutique du S.I.D.A.,
caractérisé en ce qu'il consiste:

- à multiplier des particules HIV actives dans un milieu de culture ayant une source de phosphore qui comprend l'isotope radioactif de phosphore $^{32}_{15}$P ;
- à séparer et à isoler les particules HIV ainsi cultivées dans le milieu de culture sous des conditions qui assurent la préservation d'au moins 50 % ou plus de l'antigène viral gp 120 ; et
- à stocker les particules HIV isolées, dont le génome RNA comprend au moins un groupe acide phosphorique diester dont le phosphore comprend $^{32}_{15}$P, pendant une période de plus de deux mois à une température égale ou inférieure à moins 70°C ; et
- à confectionner ensuite un vaccin prêt à être appliqué à partir de ces particules HIV.

5. Procédé suivant la revendication 4,
caractérisé en ce que,
les particules HIV inactivées utilisées pour la confection du vaccin prêt à être appliqué sont des virions HIV complets.

6. Procédé suivant la revendication 4,
caractérisé en ce que,
les particules HIV sont multipliées dans un milieu de culture dont la radioactivité obtenue par addition de $^{32}_{15}$P est d'au moins 5 µCi (Microcurie) par 1 ml de milieu de culture.

7. Procédé suivant la revendication 4,
caractérisé en ce que,
particules HIV sont multipliées dans un milieu de culture dont la radioactivité obtenue par addition de $^{32}_{15}$P est de 5 à 200 µCi par 1 ml de milieu de culture.

8. Procédé suivant la revendication 4,
caractérisé en ce que,
les particules HIV sont multipliées dans un milieu de culture qui comprend au moins 1 isotope $^{32}_{15}$P radioactif sur 300.000 isotopes P non-radioactifs.

9. Procédé suivant la revendication 4,
caractérisé en ce que,
les particules HIV sont multipliées dans un milieu de culture qui comprend au moins 1 isotope $^{32}_{15}$P radioactif sur 30.000 isotopes P non-radioactifs.

**10.** Procédé suivant la revendication 4,
<u>caractérisé en ce que,</u>
les particules HIV sont multipliées dans un milieu de culture qui comprend au moins 1 isotope $^{32}_{15}$P radioactif sur 10.000 isotopes P non-radioactifs.

**11.** Procédé suivant une des revendications 4 à 10,
<u>caractérisé en ce que,</u>
les particules HIV ainsi formées et isolées dont le génome RNA comprend au moins quelques groupes acide phosphorique diester dont le phosphore comprend $^{32}_{15}$P, sont stockées à la température égale à celle de l'azote liquide (moins 170°C).

**12.** Procédé suivant la revendication 11,
<u>caractérisé en ce que,</u>
les particules HIV sont multipliées dans un milieu de culture qui comprend au moins 4 isotopes $^{32}_{15}$P radioactifs sur 10.000 isotopes P non-radioactifs ; et que
les particules HIV ainsi formées et isolées sont stockées pendant au moins 4 mois à la température égale à celle de l'azote liquide.

**13.** Procédé suivant la revendication 11,
<u>caractérisé en ce que,</u>
les particules HIV sont multipliées dans un milieu de culture qui comprend au moins 3 isotopes $^{32}_{15}$P radioactifs sur 10.000 isotopes P non-radioactifs ; et que
les particules ainsi formées et isolées sont stockées pendant au maximum 6 mois à la température égale à celle de l'azote liquide.

**14.** Procédé suivant la revendication 4,
<u>caractérisé en ce que,</u>
les particules HIV sont multipliées dans un milieu de culture sous des conditions assurant que tout génome HIV individuel absorbe au moins 1 à 4 isotopes $^{32}_{15}$P radioactifs.

**15.** Procédé suivant la revendication 14,
<u>caractérisé en ce que,</u>
les particules HIV inactivées par incorporation de $^{32}_{15}$P sont stockées pendant 4 à 6 mois à la température égale à celle de l'azote liquide.

**16.** Procédé suivant une des revendications 4 à 15,
<u>caractérisé en ce que,</u>
ces particules HIV sont multipliées dont la structure génomique présente au moins un certain niveau de polymorphisme.

Fig.1

EP 0 745 609 B1

Fig.2

Fig.3

Fig.4

Fig.5